# EUROPÄISCHE PATENTSCHRIFT

(11) **EP 1 148 823 B1**
(45) Veröffentlichungstag und Bekanntmachung des Hinweises auf die Patenterteilung: **04.10.2006**
(21) Anmeldenummer: 00901019.0
(22) Anmeldetag: 31.01.2000
(51) Int. Cl.: A61B 17/16

(54) **VORRICHTUNG ZUR GEWINNUNG VON KNOCHENSPÄNEN**
DEVICE FOR REMOVING BONE GRAFTS
DISPOSITIF POUR PRELEVER DES COPEAUX D'OS

(30) Priorität: 02.02.1999 DE 29901723 U
(43) Veröffentlichungstag der Anmeldung: 31.10.2001
(73) Patentinhaber: Synthes AG Chur, 7002 Chur (CH)
(72) Erfinder: STEINER, Béatrice, CH-6330 Cham (CH); HEHLI, Markus, CH-7276 Frauenkirch (CH); AEBI, Max. McGill Univ., Montreal, Quebec H3A 1A1 (CA); STEFFEN, Thomas, Montreal, DC H2Y 2B7 (CA)
(74) Vertreter: Lusuardi, Werther
(86) Internationale Anmeldenummer: PCT/CH2000/000046
(87) Internationale Veröffentlichungsnummer: WO 2000/045712

(56) Entgegenhaltungen:
- WO-A-96/39956
- WO-A-97/16118
- WO-A-97/38635
- WO-A-97/39685
- US-A- 4 646 738
- US-A- 5 403 317
- US-A- 5 556 399
- US-A- 5 569 284

## Beschreibung

Die Erfindung bezieht sich auf eine Vorrichtung zur Gewinnung von Knochenspänen gemäss dem Oberbegriff des Patentanspruchs 1.

Das Implantieren von körpereigenem Knochenmaterial bleibt die effizienteste Behandlungsmethode bei Nichtheilen eines gebrochenen Knochens, Pseudoarthrosis und zur Optimierung der Erfolgsrate bei Arthrodesis. Die Verwendung von körpereigenem Knochenmaterial ist sicherer und wirksamer als die Verwendung künstlich hergestellter Hydroxyapatit-Materialien oder körperfremder Knochenspäne, bedingt jedoch einen zusätzlichen Eingriff am Körper des Patienten. Dies kann durch ein begrenztes Eindringen und durch den Gebrauch einer zylindrischen Nadel, wie sie zur Entfernung von Knochenmaterial für Diagnosezwecke verwendet wird, minimiert werden. Die Technik ist jedoch kompliziert und gefährlich da keine genaue Kontrolle gewährleistet ist. Meist wird deshalb die Spongiosa durch einen grösseren Hautschnitt und aus einer grossen Öffnung am Beckenrand herausgemeisselt. Spezielle Knochenspan-Sammelinstrumente gestatten eine sichere und rasche Gewinnung von körpereigenen Knochenspänen durch einen kleinen Hauteinschnitt, was die Unannehmlichkeiten und Verletzungen des Patienten minimiert. Diese Vorrichtungen entfemen zuverlässig das Knochenmaterial und können mit einer Bohrmaschine angewendet werden, wodurch eine grössere Menge und eine bessere Kontrollmöglichkeit gewonnen werden sowie ein versehentliches Durchstossen durch die Kortikalis minimiert wird. Diese sichere und wirksame Technik ermöglicht, körpereigene Knochenspäne für Fusionen, Pseudoarthrosis und Knochenbrüche mit einer minimalen Verletzung des Spenders zu gewinnen. Die Entfernung der Knochenspäne am Körper des Patienten wird üblicherweise am Beckenknochen vorgenommen. Ebenfalls brauchbares Knochenmaterial lässt sich proximal an der Ulna oder distal am Radius gewinnen.

Eine Methode und eine Vorrichtung zur Gewinnung von Gewebe ist aus der US 5,403,317 BONUTTI bekannt. Diese bekannte Erfindung umfasst eine Vorrichtung zur perkutanen Gewebegewinnung und besteht aus einem flexiblen Bohrerschaft und Mitteln zum Antrieb des Schaftes. Zum Herausschneiden von Gewebefragmenten aus dem Gewebe ist vorne am Schaft eine Schneidspitze angebracht. Die Gewebefragmente werden während des Schneidvorganges mittels eines Unterdruckes durch den Schaft zu einem Ort ausserhalb des Körpers gesaugt.

Nachteilig bei dieser bekannten Vorrichtung ist die Förderung der Knochenspäne durch einen mit einem Unterdruck beaufschlagten Schlauch von der Schneideinrichtung weg zu einem Filter oder anderen Abscheideeinrichtung. Die dadurch entstehenden langen Förderwege für die Knochenspäne bedingen einen grossen Unterdruck am vom Schneidkopf entfernten Ende der Förderleitung und bieten vor allem an Krümmungen der Förderleitung Möglichkeit zur unerwünschten Ablagerung von Knochenspänen innerhalb der Förderleitung.

Hier will die Erfindung Abhilfe schaffen. Der Erfindung liegt die Aufgabe zugrunde, eine Vorrichtung zu entwickeln, wo die Förderstrecke für die Knochenspäne zwischen dem Ort der Gewinnung und dem Sammelbehälter möglichst kurz ist und die Kochenspäne in einem direkt mit dem Bohrwerkzeug verbundenen Behälter gesammelt werden.

Die Erfindung löst die gestellte Aufgabe mit einer Vorrichtung zur Gewinnung von Knochenspänen, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass die erfindungsgemässe Vorrichtung einen kompakten Aufbau aufweist, Schneidwerkzeug, Antriebsmittel und Sammelbehälter in einer manuell frei bewegbaren Einheit verbunden sind und durch die Anordnung des Sammelbehälters eine hohe Saugleistung der Vorrichtung ermöglicht wird.

Die erfindungsgemässe Vorrichtung umfasst einen hohlzylindrischen . Schneidkopf, welcher verschieden gestaltete Bohrspitzen und Schneidkanten aufweisen kann, einen hohlzylindrischen Schaft mit Mitteln zum Einspannen des Schaftes in eine Antriebsvorrichtung, und eine Antriebsvorrichtung, welche beispielsweise aus einer Universalbohrmaschine bestehen kann. Die hohlzylindrische Ausgestaltung des Schneidkopfes und des Schaftes ermöglicht das Absaugen der vom Schneidkopf abgetragenen Knochenspäne aus der Spongiosa durch die Bohrung im Innern der Hohlzylinder. Zum Sammeln der Kochenspäne ist an der Antriebsvorrichtung ein Behälter so angebracht, dass der hohlzylindrische Schaft in den Behälter mündet. Zum Absaugen der Knochenspäne ist am Behälter ein Stutzen für den Anschluss eines Vakuumschlauches angebracht. Durch das so angelegte Vakuum werden die Knochenspäne durch eine oder mehrere Durchgangsöffnungen im Schneidkopf in die Bohrung im Schaft gesaugt und von dort durch den gesamten Schaft hindurch in den Behälter gefördert. Damit die Knochenspäne nicht in den Vakuumschlauch geraten, ist im Behälter eine Abscheidevorrichtung zum Abscheiden der Knochenspäne aus dem Luftstrom angebracht. Dieser Abscheider kann als Filter, Sieb, Prallplatte oder Zyklon ausgeführt sein.

In einer bevorzugten Ausführungsform umfasst die erfindungsgemässe Vorrichtung einen bezüglich Torsion und/oder Biegung elastisch verformbaren Schaft. Diese Verformbarkeit lässt sich durch eine Ausführung des Schaftes als spiralförmig gewickeltes Metallblechband, als mit einer Drahtarmierung verstärkter Kunststoff- oder Gummischlauch oder auch als Metallrohr mit balgartiger Seitenwand herstellen. Zur Abdichtung der unter Vakuum stehenden Bohrung im Schaft des Werkzeuges wird vorzugsweise in diese Bohrung ein Gummi- oder Kunststoffschlauch eingezogen. Die elastische Verformbarkeit des Schaftes und ein nicht zu scharfkantig ausgebildeter Schneidkopf gestatten ein Ausräumen der Spongiosa zwischen der Kortikalis, ohne dadurch die härtere Kortikalis zu schneiden oder durch Letztere hindurchzubrechen.

Die Verbindung zwischen Schneidkopf und Schaft ist als lösbare oder feste Verbindung denkbar, wobei eine lösbare Verbindung einen kleineren Werkzeugsatz ermöglicht. Als lösbare Verbindung sind Schraubverbindungen, radiale Stiftschrauben oder radiale Stiftverbindungen möglich.

Die Bohrspitze des Schneidkopfes ist vorzugsweise als Sektor einer Kugelkalotte mit einer Schneidkante ausgeführt. Andere Ausführungsformen der Bohrspitze sind als Kegelsektoren mit Schneidkanten oder als hohlzylindrische Fräser mit stirnseitigen Schneidezähnen denkbar.

Eine spezielle Ausführungsform des Schneidkopfes besteht darin, dass die Bohrspitze des Schneidkopfes kugelkalottenförmig mit mindestens zwei koaxial und radial zur Längsachse sich in den Hohlraum erstreckenden Durchgangsöffnungen ausgebildet ist, wobei an den Kanten der Durchgangsöffnungen Schneidkanten zum Abtragen von Knochenspänen angebracht sind und die abgetragenen Knochenspäne durch die Durchgangsöffnungen in den Hohlraum des Schneidkopfes förderbar sind.

In einer bevorzugten Anwendungsform umfasst das Vakuum einen Druckbereich von ungefähr 0 bar bis 1 bar, vorzugsweise jedoch einen Druckbereich von 0,2 bar bis 0,8 bar.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine schematische Darstellung einer Ausführungsform der erfindungsgemässen Vorrichtung;
Fig. 2 eine perspektivische Darstellung des Schneidkopfes mit dem flexiblen Schaft einer Ausführungsform der erfindungsgemässen Vorrichtung;
Fig. 3 eine Ansicht des Schneidkopfes mit dem flexiblen Schaft einer Ausführungsform der erfindungsgemässen Vorrichtung; und
Fig. 4 eine schematische Darstellung einer weiteren Ausführungsform der erfindungsgemässen Vorrichtung.

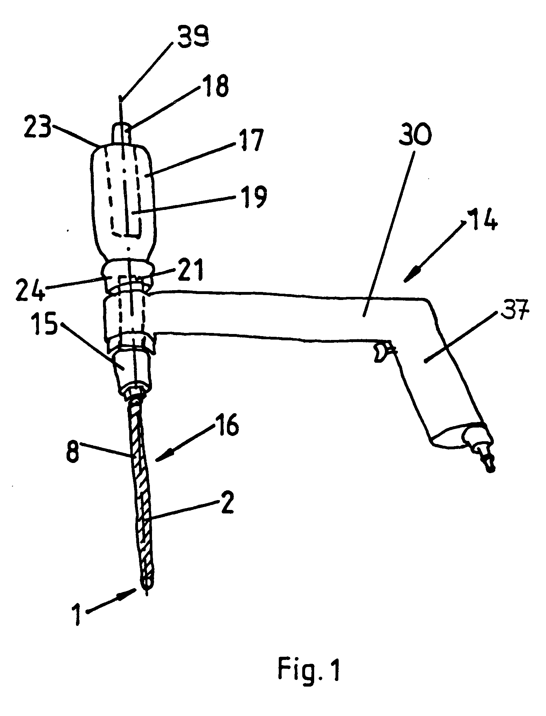

In Fig. 1 ist eine bevorzugte Ausführungsform der erfindungsgemässen Vorrichtung dargestellt. Das Schneidwerkzeug 16, welches zur Gewinnung der Knochenspäne dient, besteht aus einem Schneidkopf 1 mit einem sich entlang einer Längsachse 2 erstreckenden hohlzylindrischen Schaft 8. Dieser Schaft 8 ist in Einspannmitteln 15 einer als Antriebsmittel 14 dienenden Universalbohrmaschine 30 axial und rotativ fixiert. Die Universalbohrmaschine 30 ist mittels eines Handgriffes 37 als komplette Einheit mit Schneidwerkzeug 16 und Behälter 17 manueii frei bewegbar. Durch die Antriebsmittel 14 wird dem Schaft 8 mit dem Schneidkopf 1 eine Rotationsbewegung um die Längsachse 2 aufgeprägt, wodurch sich der Schneidkopf 1 in den Knochen bohrt und die zu sammelnden Knochenspäne abträgt. Der Schaft 8 ist vom Schneidkopf 1 bis zu seinem vom Schneidkopf 1 entfernten Ende 21 hohlzylindrisch ausgeführt, so dass die Knochenspäne entlang der gesamten Länge des Schaftes 8 förderbar sind. Ebenfalls an den Antriebsmitteln 14 angebracht ist ein Behälter 17 für die Sammlung der Knochenspäne. Der Behälter 17 ist koaxial zur Längsachse 2 mit seinem vorderen Ende 24 so an den Antriebsmitteln 14 lösbar befestigt, dass das vom Schneidkopf 1 entfernte Ende 21 des Schneidwerkzeuges 16 gegenüber der Umgebung luftdicht in den Behälter 17 mündet. Dieser gegenüber der Umgebung luftdichte Abschluss des Schneidwerkzeuges 16 in der Behälteröffnung lässt sich durch eine im wesentlichen spielfreie Lagerung des hinteren Endes 21 in der Behälteröffnung oder durch Einfügen einer Dichtung, beispielsweise einer O-Ringdichtung am hinteren Ende 21 oder in der Behälteröffnung erreichen. An seinem vom Schaft 8 entfernten Ende 23 ist der Behälter 17 mit einem Stutzen 18 versehen, woran sich ein Vakuumschlauch (nicht gezeichnet) anschliessen lässt. Durch das Vakuum im Schlauch wird der Behälter 17 ebenfalls evakuiert, wodurch im Innern des hohlzylindrischen Schaftes 8 ein Unterdruck entsteht und somit die vom Schneidkopf 1 abgetragenen Knochenspäne durch das Innere des Schaftes 8 gesaugt werden und in den Behälter 17 gelangen, wo sie dann in der Folge gesammelt werden können. Damit die Knochenspäne nicht durch das Vakuum mit in den Schlauch gerissen werden, ist im Behälter 17 eine Abscheidevorrichtung 19, welche in der bevorzugten Ausführungsform als Sieb ausgestaltet ist, so angebracht, dass die Knochenspäne nicht durch den Stutzen 18 austreten können.

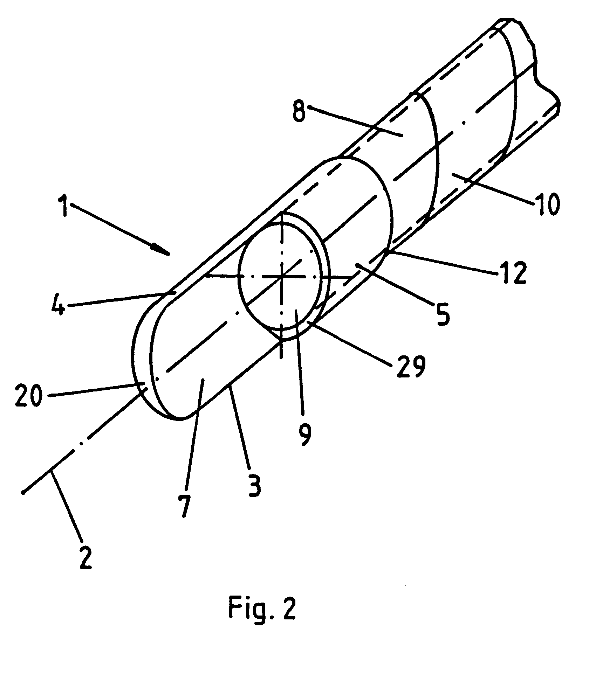

Fig. 2 zeigt eine Ausführungsform des Schneidkopfes 1. Der Schneidkopf 1 ist als Hohlzylinder mit einer Längsachse 2 und einer Bohrspitze 20 ausgeführt und umfasst einen vorderen an die Bohrspitze 20 anschliessenden Abschnitt 4 und einen hinteren von der Bohrspitze 20 entfernten Abschnitt 5. Der vordere Abschnitt 4 besteht aus einem Hohlzylinder mit einer als Kugelkalottensektor ausgebildeten Bohrspitze 20, wobei die im Querschnitt rechtwinklig zur Längsachse 2 betrachtete Seitenwand des vorderen Abschnitts 4 nur einen Kreisringsektor einschliesst, so dass eine radial zum hohlzylindrischen Teil und axial zur Bohrspitze 20 verlaufende Durchgangsöffnung 7 entsteht. Die Seitenwand des vorderen Abschnittes 4 ist von der Bohrspitze 20 bis zum hinteren Abschnitt 5 gegen die Durchgangsöffnung 7 hin als Schneidkante 3 ausgebildet. Wird der rotierende Schneidkopf 1 in den Knochen gebohrt, so werden durch die Schneidkanten 3 Knochenspäne abgetragen und gelangen durch die Durchgangsöffnung 7 in den Hohlraum 9 des Schneidkopfes 1 und werden von dort durch die Bohrung 10 im Schaft 8 durch das Vakuum abgesaugt.

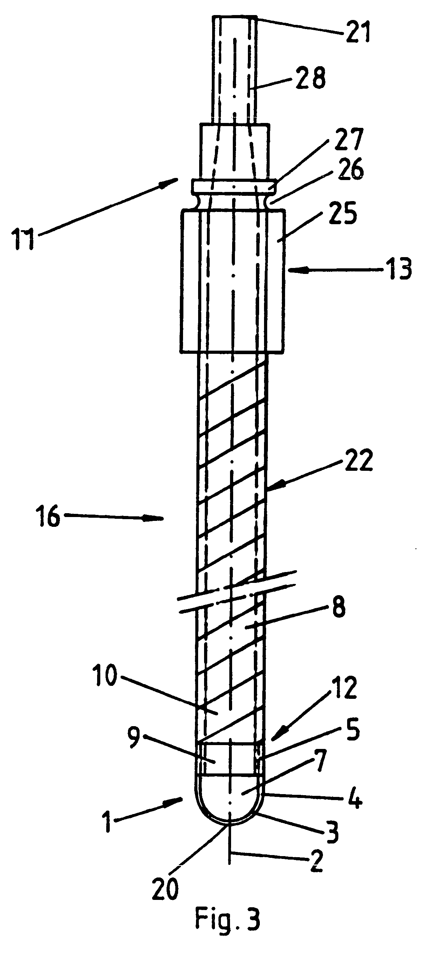

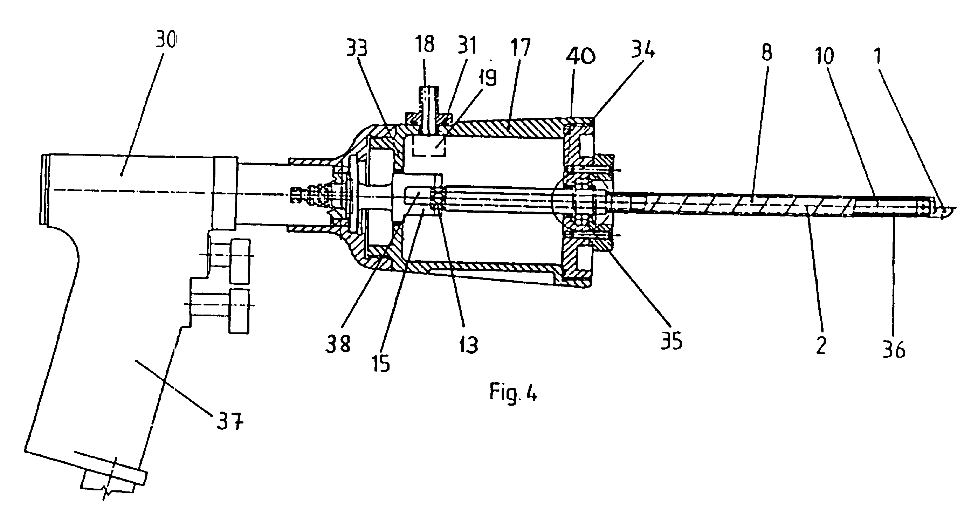

In Fig. 3 ist das Werkzeug 16 mit Schneidkopf 1 und Schaft 8 dargestellt. Der Schaft 8 umfasst einen bezüglich Torsion und/oder Biegung elastisch verformbaren Teil 22 und einen mit Mitteln 13 zur Aufnahme eines Drehmomentes versehenen, vom Schneidkopf 1 entfernten Teil 11. Die Mittel 13 bestehen aus einem Abschnitt 25 mit Aussensechskant und einem daran anschliessenden zylindrischen Abschnitt 27 mit einer Nute 26. Die beiden Abschnitte 25 und 27 lassen sich in entsprechende Einspannmittel 15 (Fig. 1) an einem Antriebsmittel 14 (Fig. 1) einspannen, wobei der Schaft 8 mittels der Nute 26 axial und durch den Aussensechskant rotativ im Einspannmittel 15 (Fig. 1) lösbar fixierbar ist. Die Bohrung 10 im hohlzylindrischen Schaft 8 durchdringt den Schaft 8 in Richtung der Längsachse 2 vom Schneidkopf 1 bis zu dem vom Schneidkopf 1 entfernten Ende 21 des Schaftes 8, so dass die vom Schneidkopf 1 abgetragenen Knochenspäne entlang der Längsachse 2 durch das ganze Werkzeug 16 förderbar sind. Zur Fixation des Schneidkopfes 1 am Schaft 8 sind Feststellschrauben oder beispielsweise auch Federstifte zwischen Schaft 8 und Schneidkopf 1 denkbar. Der elastisch verformbare Teil 22 des Schaftes 8 ist aus einem spiralförmig gewickelten Metallstreifen gefertigt, wobei in der Bohrung 10 ein Gummi- oder Kunststoffschlauch 36 (Fig. 4) eingelegt ist, welcher gegenüber der Umgebung einen luftdichten Abschluss in der Bohrung im Schlauch 36 gewährleistet. Gegen das vom Schneidkopf 1 entfernte Ende 21 des Schaftes 8 ist dieser anschliessend an die Mittel 11 zur Aufnahme eines Drehmomentes wieder als hohlzylindrischer Teil 28 ausgebildet, so dass eine ebenfalls gegenüber der Umgebung luftdichter Abschluss dieses Teils 28 bei der Einmündung in den Behälter 17 (Fig. 1) möglich ist.

In Fig. 4 ist eine weitere bevorzugte Ausführungsform der erfindungsgemässen Vorrichtung dargestellt. Diese hier dargestellte Ausführungsform der erfindungsgemässen Vorrichtung unterscheidet sich von der in Fig. 1 dargestellten Ausführungsform nur darin, dass das Schneidwerkzeug 16 durch den koaxial zur Längsachse 2 angeordneten Behälter 17 durchgeht und die Mittel 13 zur Aufnahme eines durch die Universalbohrmaschine 30 abgegebenen Drehmomentes im Bereich des vom Schneidkopf 1 entfernten Behälterbodens 33 mit der Universalbohrmaschine 30 lösbar verbunden sind. Der Behälter 17 ist mit seinem Behälterboden 33 an der Universalbohrmaschine 30 lösbar befestigt. Anstelle eines Behälterdeckels ist ein Lagergehäuse 34 im Behälter 17 angebracht, worin das Werkzeug 16 bezüglich seiner Rotationsbewegung um die Längsachse 2 beispielsweise mittels Kugellager 35 gelagert ist. Auch bei dieser Ausführungsform lässt sich ein luftdichter Abschluss des Schneidwerkzeuges 16 im Lagergehäuse 34 gegenüber der Umgebung durch durch Einfügen einer Dichtung, beispielsweise einer O-Ringdichtung 40 zwischen Schneidwerkzeug 16 und Lagergehäuse 34 erreichen. Zudem ist der Stutzen 18 für den Anschluss eines Vakuumschlauches an der Seitenwand des Behälters 17 angebracht. Zur Abdichtung des flexiblen Schaftes 8 ist in dessen Bohrung 10 ein Gummi- oder Kunststoffschlauch 36 entlang der Längsachse 2 eingeführt. Die vom Schneidkopf 1 abgetragenen Knochenspäne werden durch das Vakuum durch die das Werkzeug 16 koaxial zur Längsachse 2 durchdringende Bohrung 10 bis zu dem vom Schneidkopf 1 entfernten Ende 21 des Werkzeuges 16 gefördert und dort durch Öffnungen 38 in den Einspannmitteln 15 in den Behälter 17 gesaugt. Damit die Knochenspäne nicht durch den Stutzen 18 mit in den Vakuumschlauch (nicht gezeichnet) gerissen werden, ist im Behälter 17 eine Abscheidevorrichtung 19, welche bevorzugt als Sieb ausgestaltet ist, angebracht.

## Patentansprüche

1. Vorrichtung zur Gewinnung von Knochenspänen, welche
A) ein rotierbares, hohlzylindrisches Schneidwerkzeug (16) mit einer Längsachse (2), einem Schneidkopf (1) und einem an den Schneidkopf (1) anschliessenden, konzentrisch zur Längsachse (2) angeordneten longitudinalen Schaft (8);
B) Antriebsmittel (14), welche mit einem Handgriff (37) versehen sind und mittels welcher dem Schneidwerkzeug (16) mit dem Schneidkopf (1) eine Rotationsbewegung um die Längsachse (2) aufprägbar ist; und
C) einen unter Vakuum stehenden, mit dem Schaft (8) verbindbaren Behälter (17) mit einer Zentralachse (39) umfasst; wobei
D) das Schneidwerkzeug (16) eine in Richtung der Längsachse (2) durchgehende Bohrung (10) aufweist und der Schneidkopf (1) mit mindestens einer Durchgangsöffnung (7) versehen ist, so dass die vom Schneidkopf (1) spanabhebend abgetragenen Knochenspäne durch die Bohrung (10) förderbar sind; und
E) auch während der spanabhebenden Gewinnung von Knochenspänen die Knochenspäne mittels des Vakuums im Behälter (17) durch die Bohrung (10) vom Schneidkopf (1) in den Behälter (17) förderbar sind,
**dadurch gekennzeichnet, dass**
F) Schneidwerkzeug (16), Behälter (17) und Antriebsmittel (14) zu einer mittels des Handgriffes (37) manuell frei bewegbaren Vorrichtung verbunden sind, und
G) der Behälter (17) so am Antriebsmittel (14) lösbar angebracht ist, dass die Zentralachse (39) konzentrisch zur Längsachse (2) verläuft.

2. Vorrichtung nach Anspruch 1, **dadurch gekennzeichnet, dass** das vom Schneidkopf (1) entfernte Ende (21) des Schneidwerkzeuges (16) in den Behälter (17) mündet.

3. Vorrichtung nach Anspruch 2, **dadurch gekennzeichnet, dass** der Behälter (17) relativ zur Längsachse (2) stillsteht und das vom Schneidkopf (1) entfernte Ende (21) des rotierenden hohlzylindrischen Schneidwerkzeuges (16) in den Behälter (17) mündet.

4. Vorrichtung nach Anspruch 2 oder 3, **dadurch gekennzeichnet, dass** das vom Schneidkopf (1) entfernte Ende (21) des rotierenden hohlzylindrischen Schneidwerkzeuges (16) so in den Behälter (17) mündet, dass der Übergang zwischen Schneidwerkzeug (16) und Behälter (17) gegenüber der Umgebung luftdicht abgeschlossen ist.

5. Vorrichtung nach Anspruch 4, **dadurch gekennzeichnet, dass** das vom das vom Schneidkopf (1) entfernte Ende (21) des rotierenden hohlzylindrischen Schneidwerkzeuges (16) im wesentlichen spielfrei in den Behälter (17) mündet.

6. Vorrichung nach Anspruch 4, **dadurch gekennzeichnet, dass** das vom Schneidkopf (1) entfernte Ende (21) des rotierenden hohlzylindrischen Schneidwerkzeuges (16) eine Dichtung umfasst und der Behälter (17) eine Behälteröffnung umfasst, so dass die Dichtung den ringförmigen Spalt zwischen dem Ende (21) des Schneidwerkzeuges (16) und der Behälteröffnung abdichtet und damit der Übergang zwischen Schneidwerkzeug (16) und Behälter (17) gegenüber der Umgebung luftdicht abgeschlossen ist.

7. Vorrichtung nach Anspruch 3, **dadurch gekennzeichnet, dass** der Behälter (17) ein Lagergehäuse (34) umfasst, worin das Schneidwerkzeug (16) konzentrisch zur Längsachse (2) und um diese rotierbar gelagert ist.

8. Vorrichtung nach Anspruch 7, **dadurch gekennzeichnet, dass** das Lagergehäuse (34) ein Kugellager (35) umfasst, worin das Schneidwerkzeug (16) konzentrisch zur Längsachse (2) und um diese rotierbar gelagert ist.

9. Vorrichtung nach Anspruch 7 oder 8, **dadurch gekennzeichnet, dass** das Lagergehäuse (34) eine Dichtung umfasst, wodurch die Lagerung des Schneidwerkzeuges (16) im Lagergehäuse (34) gegenüber der Umgebung luftdicht abgeschlossen ist.

10. Vorrichtung nach Anspruch 9, **dadurch gekennzeichnet, dass** die Dichtung eine O-Ringdichtung (40) ist.

11. Vorrichtung nach einem der Ansprüche 1 bis 10, **dadurch gekennzeichnet, dass** am Behälter (17) ein Stutzen (18) für den Anschluss eines Vakuumschlauches angebracht ist.

12. Vorrichtung nach einem der Ansprüche 1 bis 11, **dadurch gekennzeichnet, dass** der Behälter (17) eine Abscheidevorrichtung (19) zur Abscheidung der Knochenspäne aus dem Luftstrom umfasst.

13. Vorrichtung nach Anspruch 12, **dadurch gekennzeichnet, dass** die Abscheidevorrichtung (19) aus einem Filter besteht.

14. Vorrichtung nach einem der Ansprüche 1 bis 13, **dadurch gekennzeichnet, dass** der Schaft (8) auf einem an den Schneidkopf (1) anschliessenden Teil (22) bezüglich Torsion und/oder Biegung elastisch ist.

15. Vorrichtung nach Anspruch 14, **dadurch gekennzeichnet, dass** der Schaft (8) aus einem spiralförmig gewickelten Metallband gefertigt ist.

16. Vorrichtung nach einem der Ansprüche 1 bis 15, **dadurch gekennzeichnet, dass** in der Bohrung (10) des Schaftes (8) entlang der Längsachse (2) zusätzlich ein Kunststoff- oder Gummischlauch eingeführt ist.

17. Vorrichtung nach Anspruch 14 oder 16, **dadurch gekennzeichnet, dass** der Schaft (8) aus einem Metallrohr mit balgartiger Wand besteht.

18. Vorrichtung nach einem der Ansprüche 1 bis 17, **dadurch gekennzeichnet, dass** der Schneidkopf (1) hohlzylindrisch gestaltet ist und einen sich entlang der Längsachse (2) erstreckenden Hohlraum (9), einen mit einer Bohrspitze (20) und mit mindestens einer Schneidkante (3) versehenen vorderen Abschnitt (4), einen hohlzylindrischen hinteren Abschnitt (5) und mindestens eine im vorderen Abschnitt (4) die Aussenwand (29) des Schneidkopfes (1) radial durchdringende Durchgangsöffnung (7) zur Förderung der durch die mindestens eine Schneidkante (3) abgetragenen Knochenspäne in den Hohlraum (9) des Schneidkopfes (1) umfasst.
und mit mindestens einer Schneidkante (3) versehenen vorderen Abschnitt (4), einen hohlzylindrischen hinteren Abschnitt (5) und mindestens eine im vorderen Abschnitt (4) die Aussenwand (29) des Schneidkopfes (1) radial durchdringende Durchgangsöffnung (7) zur Förderung der durch die mindestens eine Schneidkante (3) abgetragenen Knochenspäne in den Hohlraum (9) des Schneidkopfes (1) umfasst.

19. Vorrichtung nach Anspruch 18, **dadurch gekennzeichnet, dass** die Bohrspitze (20) des Schneidkopfes (1) als Kugelkalottensektor ausgebildet ist.

20. Vorrichtung nach Anspruch 18, **dadurch gekennzeichnet, dass** die Bohrspitze (20) des Schneidkopfes (1) kugelkalottenförmig mit mindestens einer koaxial und radial zur Längsachse (2) sich in den Hohlraum (9) erstreckenden Durchgangsöffnung (7) ausgebildet ist, wobei an den Kanten der Durchgangsöffnungen (7) Schneidkanten (3) zum Abtragen von Knochenspänen angebracht sind und die abgetragenen Knochenspäne durch die Durchgangsöffnungen (7) in den Hohlraum (9) des Schneidkopfes (1) förderbar sind.

21. Vorrichtung nach einem der Ansprüche 1 bis 20, **dadurch gekennzeichnet, dass** die Antriebsmittel (14) aus einer Universalbohrmaschine (30) bestehen.

22. Vorrichtung nach einem der Ansprüche 1 bis 21, **dadurch gekennzeichnet, dass** der Schaft (8) durchgehend hohlzylindrisch ausgeführt ist, Mittel (13) zur Aufnahme eines Drehmomentes, welches eine Rotation des Schaftes (8) um die Längsachse (2) verursacht, aufweist und an seinem am Schneidkopf (1) anschliessbaren Ende (12) so an den hinteren Abschnitt (5) anschliessbar ist, dass die Bohrung (10) des hohlzylindrischen Schaftes (8) mit dem Hohlraum (9) fluchtend ausrichtbar ist und vom Schaft (8) das Drehmoment auf den Schneidkopf (1) übertragbar ist.

23. Vorrichtung nach Anspruch 22, **dadurch gekennzeichnet, dass** die Antriebsmittel (14) Einspannmittel (15) zur rotativen und axialen Fixierung der Mittel (13) am Schaft (8) umfassen.

## Claims

1. A device for removing bone grafts, comprising
A) a rotating hollow cylindrical cutting tool (16) with a longitudinal axis (2), a cutting head (1) and a longitudinal shaft (8) connected to the cutting head (1) and arranged concentrically with the longitudinal axis (2);
B) drive means (14) which are provided with a handle (37) and by means of which a rotational motion about the longitudinal axis (2) can be applied to the cutting tool (16) with the cutting head (1); and
C) a container (17) that can be connected to the shaft (8) and is under vacuum and has a central axis (39); whereby
D) the cutting tool (16) has a continuous bore (10) in the direction of the longitudinal axis (2), and the cutting head (1) is provided with at least one through-hole (7), so that the bone grafts removed by the cutting head (1) can be conveyed through the bore (10); and
E) the bone grafts can also be conveyed into the container (17) from the cutting head (1) through the bore (10) by means of the vacuum in the container (17) even during the cutting and harvesting of the bone grafts;
**characterized in that**
F) the cutting tool (16), container (17) and drive means (14) are connected to a device that is freely movable manually by the handle (37), and
G) the container (17) is detachably mounted on the drive means (14) in such a way that the central axis (39) is concentric with the longitudinal axis (2).

2. A device according to Claim 1, **characterized in that** the end (21) of the cutting tool (16) at a distance from the cutting head (1) opens into the container (17).

3. A device according to Claim 2, **characterized in that** the container (17) is stationary relative to the longitudinal axis (2), and the end (21) of the rotating hollow cylindrical cutting tool (16) at a distance from the cutting head (1) opens into the container (17).

4. A device according to Claim 2 or 3, **characterized in that** the end (21) of the rotating hollow cylindrical cutting tool (16) at a distance from the cutting head (1) opens into the container (17) in such a way that the transition between the cutting tool (16) and the container (17) is sealed airtight with respect to the environment.

5. A device according to Claim 4, **characterized in that** the end (21) of the rotating hollow cylindrical cutting tool (16) at a distance from the cutting head (1) opens into the container (17) with essentially no play.

6. A device according to Claim 4, **characterized in that** the end (21) of the rotating hollow cylindrical cutting tool (16) at a distance from the cutting head (1) comprises a gasket, and the container (17) comprises a container opening such that the gasket seals the annular gap between the end (21) of the cutting tool (16) and the container opening and thus forms an airtight seal for the transition between the cutting tool (16) and the container (17) with respect to the environment.

7. A device according to Claim 3, **characterized in that** the container (17) comprises a bearing housing (34) wherein the cutting tool (16) is mounted to be concentric with the longitudinal axis (2) so it can rotate about the latter.

8. A device according to Claim 7, **characterized in that** the bearing housing (34) comprises a ball bearing (35) wherein the cutting tool (16) is mounted so it is concentric with the longitudinal axis (2) and can rotate about the latter.

9. A device according to Claim 7 or 8, **characterized in that** the bearing housing (34) comprises a gasket so that the cutting tool (16) is provided with an airtight seal with respect to the environment in the bearing housing (34).

10. A device according to Claim 9, **characterized in that** the gasket is an O-ring gasket (40).

11. A device according to one of Claims 1 through 10, **characterized in that** a connection (18) is provided on the container (17) for connecting a vacuum line.

12. A device according to one of Claims 1 thorugh 11, **characterized in that** the container (17) has a separation device (19) for separating the bone grafts from the air stream.

13. A device according to Claim 12, **characterized in that** the separation device (19) consists of a filter.

14. A device according to one of Claims 1 through 13, **characterized in that** the shaft (8) on a part (22) which is connected to the cutting head (1) is elastic with respect to torsion and/or bending.

15. A device according to Claim 14, **characterized in that** the shaft (8) is made of a metal strip wound in a spiral.

16. A device according to one of Claims 1 through 15, **characterized in that** a rubber or plastic tube is also inserted into the bore (10) of the shaft (8) along the longitudinal axis (2).

17. A device according to Claim 14 or 16, **characterized in that** the shaft (8) consists of a metal tube with a bellows-like wall.

18. A device according to one of Claims 1 through 17, **characterized in that** the cutting head (1) is designed as a hollow cylinder and comprises a hollow space (9) extending along the longitudinal axis (2), a distal section (4) which is provided with a drill tip (20) and with at least one cutting edge (3), a hollow cylindrical proximal section (5) and at least one through-hole (7) which passes through the outside wall (29) of the cutting head (1) radially in the distal section(4) for conveying bone grafts removed by the minimum of one cutting edge (3) into the hollow space (9) of the cutting head (1).

19. A device according to Claim 18, **characterized in that** the drill tip (20) of the cutting head (1) is designed as a sector of a universal ball joint.

20. A device according to Claim 18, **characterized in that** the drill tip (20) of the cutting head (1) is designed as a sector of a universal ball joint with at least one through-hole (7) which extends coaxially and radially with the longitudinal axis (2) in the hollow space (9), with cutting edges (3) for removing bone grafts being mounted on the edges of the through-holes (7) and the bone grafts removed being conveyable through the through-holes (7) into the hollow space (9) of the cutting head (1).

21. A device according to one of Claims 1 through 20, **characterized in that** the drive means (14) consist of a universal drilling machine (30).

22. A device according to one of Claims 1 through 21, **characterized in that** the shaft (8) is designed to be a hollow cylinder and has means (13) for absorbing a torque which causes rotation of the shaft (8) about the longitudinal axis (2) and can be connected to the proximal section (5) on its end (12) connected to the cutting head (1) such that the bore (10) of the hollow cylindrical shaft (8) can be aligned with the hollow space (9) and the torque can be transmitted from the shaft (8) to the cutting head (1).

23. A device according to Claim 22, **characterized in that** the drive means (14) comprise chucking means (15) for securing the means (13) on the shaft (8) rotationally and axially.

## Revendications

1. Dispositif de prélèvement de copeaux d'os, lequel comprend
A) un outil de coupe rotatif en forme de cylindre creux (16) ayant un axe longitudinal (2), une tête coupante (1) et une tige (8) longitudinale dans le prolongement de la tête coupante (1), disposée concentriquement à l'axe longitudinal (2) ;
B) des moyens d'entraînement (14), lesquels sont pourvus d'une poignée (37) et permettent à l'outil de coupe (16) avec la tête coupante (1) d'effectuer un mouvement de rotation autour de l'axe longitudinal (2) ; et
C) un récipient (17) sous vide ayant un axe central (39), pouvant être relié à la tige (8) ; dans lequel
D) l'outil de coupe (16) présente un trou traversant (10) dans le sens de l'axe longitudinal (2) et la tête coupante (1) est pourvue d'au moins une ouverture de passage (7), de sorte que les copeaux d'os prélevés par enlèvement de copeaux avec la tête coupante (1) peuvent être évacués à travers le trou (10) ; et
E) les copeaux d'os peuvent, sous l'action du vide dans le récipient (17), être évacués à travers le trou (10) de la tête coupante (1) dans le récipient (17), même pendant le prélèvement de copeaux d'os par enlèvement de copeaux,
**caractérisé en ce que**
F) l'outil de coupe (16), le récipient (17) et les moyens d'entraînement (14) sont reliés entre eux pour former un dispositif pouvant être manipulé librement au moyen de la poignée (37), et
G) le récipient (17) est disposé de manière amovible sur les moyens d'entraînement (14) de telle manière que l'axe central (39) s'étend concentriquement à l'axe longitudinal (2).

2. Dispositif selon la revendication 1, **caractérisé en ce que** l'extrémité (21) de l'outil de coupe (16) éloignée de la tête coupante (1) débouche dans le récipient (17).

3. Dispositif selon la revendication 2, **caractérisé en ce que** le récipient (17) est fixe par rapport à l'axe longitudinal (2) et l'extrémité (21) de l'outil de coupe rotatif en forme de cylindre creux (16) éloignée de la tête coupante (1) débouche dans le récipient (17).

4. Dispositif selon la revendication 2 ou 3, **caractérisé en ce que** l'extrémité (21) de l'outil de coupe rotatif en forme de cylindre creux (16) éloignée de la tête coupante (1) débouche dans le récipient (17) de telle manière que la zone de transition entre l'outil de coupe (16) et le récipient (17) est hermétique vers l'extérieur.

5. Dispositif selon la revendication 4, **caractérisé en ce que** l'extrémité (21) de l'outil de coupe rotatif en forme de cylindre creux (16) éloignée de la tête coupante (1) débouche dans le récipient (17) essentiellement sans jeu.

6. Dispositif selon la revendication 4, **caractérisé en ce que** l'extrémité (21) de l'outil de coupe rotatif en forme de cylindre creux (16) éloignée de la tête coupante (1) comprend un joint d'étanchéité et le récipient (17) comprend une ouverture de récipient, de sorte que le joint d'étanchéité rend étanche la fente annulaire entre l'extrémité (21) de l'outil de coupe (16) et l'ouverture de récipient et que la zone de transition entre l'outil de coupe (16) et le récipient (17) est ainsi hermétique vers l'extérieur.

7. Dispositif selon la revendication 3, **caractérisé en ce que** le récipient (17) comprend un logement de palier (34), dans lequel l'outil de coupe (16) est logé concentriquement à l'axe longitudinal (2) et de manière à pouvoir tourner autour de celui-ci.

8. Dispositif selon la revendication 7, **caractérisé en ce que** le logement de palier (34) comprend un palier à billes (35), dans lequel l'outil de coupe (16) est logé concentriquement à l'axe longitudinal (2) et de manière à pouvoir tourner autour de celui-ci.

9. Dispositif selon la revendication 7 ou 8, **caractérisé en ce que** le logement de palier (34) comprend un joint d'étanchéité, si bien que le logement de l'outil de coupe (16) dans le logement de palier (34) est hermétique vers l'extérieur.

10. Dispositif selon la revendication 9, **caractérisé en ce que** le joint d'étanchéité est un joint torique (40).

11. Dispositif selon l'une quelconque des revendications 1 à 10, **caractérisé en ce qu'**un raccord (18) est disposé sur le récipient (17) pour le raccordement d'un tuyau à vide.

12. Dispositif selon l'une quelconque des revendications 1 à 11, **caractérisé en ce que** le récipient (17) comprend un dispositif de séparation (19) pour séparer les copeaux d'os du courant d'air.

13. Dispositif selon la revendication 12, **caractérisé en ce que** le dispositif de séparation (19) consiste en un filtre.

14. Dispositif selon l'une quelconque des revendications 1 à 13, **caractérisé en ce que** la tige (8), sur une partie (22) dans le prolongement de la tête coupante (1), est élastique en termes de torsion et/ou de flexion.

15. Dispositif selon la revendication 14, **caractérisé en ce que** la tige (8) est faite d'un ruban métallique enroulé en spirale.

16. Dispositif selon l'une quelconque des revendications 1 à 15, **caractérisé en ce qu'**un tuyau de matière plastique ou de caoutchouc est inséré en plus dans le trou (10) de la tige (8) le long de l'axe longitudinal (2).

17. Dispositif selon la revendication 14 ou 16, **caractérisé en ce que** la tige (8) est faite d'un tube métallique à paroi annelée.

18. Dispositif selon l'une quelconque des revendications 1 à 17, **caractérisé en ce que** la tête coupante (1) est conçue sous forme de cylindre creux et comprend un espace creux (9) s'étendant le long de l'axe longitudinal (2), un segment avant (4) pourvu d'une pointe de perçage (20) et d'au moins une arête coupante (3), un segment arrière en forme de cylindre creux (5) et au moins une ouverture de passage (7) traversant radialement la paroi externe (29) de la tête coupante (1) dans le segment avant (4) pour évacuer les copeaux d'os prélevés avec la au moins une arête coupante (3) dans l'espace creux (9) de la tête coupante (1).

19. Dispositif selon la revendication 18, **caractérisé en ce que** la pointe de perçage (20) de la tête coupante (1) est conçue sous forme de segment d'une calotte sphérique.

20. Dispositif selon la revendication 18, **caractérisé en ce que** la pointe de perçage (20) de la tête coupante (1) est conçue sous forme de segment d'une calotte sphérique avec au moins une ouverture de passage (7) s'étendant coaxialement et radialement à l'axe longitudinal (2) dans l'espace creux (9), dans lequel sont disposées des arêtes coupantes (3) sur les bords des ouvertures de passage (7) pour prélever des copeaux d'os, et les copeaux d'os prélevés peuvent être évacués à travers les ouvertures de passage (7) dans l'espace creux (9) de la tête coupante (1).

21. Dispositif selon l'une quelconque des revendications 1 à 20, **caractérisé en ce que** les moyens d'entraînement (14) consistent en une perceuse universelle (30).

22. Dispositif selon l'une quelconque des revendications 1 à 21, **caractérisé en ce que** la tige (8) est conçue sous forme de cylindre creux ininterrompu, présente des moyens (13) pour la réception d'un couple de rotation, lequel provoque une rotation de la tige (8) autour de l'axe longitudinal (2), et peut, au niveau de son extrémité (12) pouvant être reliée à la tête coupante (1), être reliée au segment arrière (5) de telle manière que le trou (10) de la tige en forme de cylindre creux (8) peut être mis en affleurement avec l'espace creux (9) et que le couple de rotation peut être transmis à la tête coupante (1) par la tige (8).

23. Dispositif selon la revendication 22, **caractérisé en ce que** les moyens d'entraînement (14) comprennent des moyen de serrage (15) pour bloquer les moyens (13) sur la tige (8) en rotation et sur l'axe.
